# EUROPEAN PATENT SPECIFICATION

(11) **EP 4 458 269 B1**
(45) Date of publication and mention of the grant of the patent: **18.11.2026**
(21) Application number: 22916292.0
(22) Date of filing: 15.09.2022
(51) Int. Cl.: A61B 5/346, A61B 5/00, A61N 1/04, A61N 1/365, A61N 1/362, A61N 1/36

(54) **ELECTROCARDIOGRAM MONITORING DEVICE COMPRISING ELECTRICAL STIMULATION FUNCTION**
ELEKTROKARDIOGRAMMÜBERWACHUNGSVORRICHTUNG MIT ELEKTRISCHER STIMULATIONSFUNKTION
DISPOSITIF DE SURVEILLANCE ÉLECTROCARDIOGRAPHIQUE COMPRENANT UNE FONCTION DE STIMULATION ÉLECTRIQUE

(30) Priority: 30.12.2021 KR 20210193231
(43) Date of publication of application: 06.11.2024
(73) Proprietor: Mezoo Co., Ltd., Wonju-si, Gangwon-do 26354 (KR)
(72) Inventor: PARK, Jung Hwan, Wonju-si Gangwon-do 26494 (KR); CHOI, Hyun Seok, Wonju-si Gangwon-do 26424 (KR); CHO, Sung Pil, Wonju-si Gangwon-do 26494 (KR)
(74) Representative: Zimmermann, Tankred Klaus
(86) International application number: PCT/KR2022/013785
(87) International publication number: WO 2023/128138

(56) References cited:
- JP-A- 2010 187 775
- JP-A- 2012 529 286
- JP-A- 2020 503 105
- KR-A- 20150 104 888
- KR-A- 20170 121 212
- US-A1- 2019 001 131
- US-A1- 2021 379 374
- US-B2- 10 391 297
- US-B2- 11 058 878

## Description

### [Technical Field]

The present invention relates to an electrocardiogram monitoring device with an electrical stimulation application function, more particularly, to an electrocardiogram monitoring device with an electrical stimulation application function, in which distortion is minimized by adapting the pattern of electrical stimulation output, as biofeedback based on the electrocardiogram measurement results, to the electrocardiogram measurement timing.

### [Background Art]

Due to the miniaturization and wearability of biometric information detection devices as well as the development of communication technologies such as 5G and IoT, remote health monitoring and medical technology, along with on-site analysis of users, are rapidly developing. Especially electrocardiogram (ECG) monitoring is mainly used to detect cardiac abnormalities such as arrhythmia, myocardial infarction, and angina pectoris. Due to its importance in life activities, portable electrocardiogram monitoring devices capable of communication are widely used in remote health or user monitoring.

An electrocardiogram is an action current resulting from the contraction and expansion of the heart muscle. It is a current generated when the action potential generated when the heart muscle contracts and relaxes spreads from the heart to the entire body. Since this current generates a potential difference depending on the body position, it may be detected and measured through two or more electrodes attached to the human skin.

Conventional electrocardiogram monitoring devices have been manufactured for the purpose of simply measuring and monitoring the user's electrocardiogram and transmitting the measured or analyzed data to a remote healthcare system or medical staff. However, when a risk to the user's heart condition is recognized based on such electrocardiogram data, it is necessary to warn the user or provide light treatment as biofeedback, which applies physical stimulation to the corresponding user. Biofeedback means measuring the body and mind responses using a special device and providing feedback in a form that may be physically perceived so that the users may control their relaxation or tension based on the measurement information.

Conventional biofeedback is mainly obtained by installing a biofeedback device, which is independent of the electrocardiogram monitoring device, in a specific space and having the user lie down or sit comfortably.

However, this conventional technology had problems in that it was difficult to provide continuous monitoring or biofeedback in daily life because it was limited in space and the user's movement or motion was limited.

In relation to this, Republic of Korea Patent Application Publication No. 2007-0097900 discloses an electrocardiogram measuring device which measures the user's electrocardiogram, determines the risk based on the electrocardiogram data, outputs a sound source file to the user to perform acoustic therapy to reduce the instability if it is determined to be mild risk according to the electrocardiogram risk, and stops the output of the sound source file and transmits an alarm sound to notify people around the user of the risk if the risk of the electrocardiogram is high. However, in the case where the risk of the electrocardiogram is light, acoustic therapy based on the output of the sound source file has a problem in that the effect of alleviating the unstable state of the user's electrocardiogram is very small.

In addition, Japanese Patent Application Publication No. 2018-531642 discloses a wearable device including a detection electrode capable of detecting heart signals and an integrated defibrillator electrode pad for electrical stimulation that can deliver a therapeutic shock to the user when a danger signal is detected in the heart. However, in case where continuous monitoring and alarm electrical stimulation are provided rather than a one-time shock using defibrillator electrode pads in situations where urgent treatment is needed, or in case where the risk is mild, i.e., where the user requires continuous low-level electrical stimulation, such as therapeutic electrical stimulation to relieve instability, the problem with this literature is that when electrocardiogram measurement is performed at the same time that electrical stimulation is applied to the user, the electrical stimulation causes distortion in the electrocardiogram signal, making it difficult to accurately monitor the electrocardiogram.

In particular, transcutaneous electrical nerve stimulation (TENS) may be used as a low-level electrical stimulation in humans. This type of electrical stimulation is mainly used to relieve pain by providing electrical stimulation to the skin and muscles. A general TENS device may adjust the frequency, pulse width, and intensity of electrical stimulation, with a preferred frequency of about 1 to 200 Hz and a pulse width of about 50 to 200 µs. However, the frequency and amplitude of this electrical stimulation are similar to those of an electrocardiogram.

Therefore, when the application of such electrical stimulation is performed together with electrocardiogram measurement, the accuracy of electrocardiogram measurement is reduced due to distortion occurring in the electrocardiogram signal. Therefore, electrical stimulation and electrocardiogram measurement are not usually performed together, and a separate device for the electrocardiogram measurement and electrical stimulation was required. Document US 2019/ 0001131 A1 discloses portable and integrated modules and devices for non-intrusive neuromodulation and monitoring of heart function of that type. The modules and devices include a monitoring unit, an electrical stimulation inducing unit, and three electrodes, such that one of the electrodes is dual-function, being controllably utilized for neuromodulation or for monitoring of heart function.

(Patent Document 1) Republic of Korea Patent Application Publication No. 2007-0097900

(Patent Document 2) Japanese Patent Application Publication No. 2018-531642

### [Disclosure]

### [Technical Problem]

Therefore, a technical object to be achieved by the present invention is to provide an electrocardiogram monitoring device with electrical stimulation function, which when performing electrocardiogram monitoring in a remote healthcare system or remote medical system, feedback for a warning or light treatment can be provided to the user based on electrocardiogram data in the form of electrical stimulation that does not affect subsequent electrocardiogram monitoring, such as distortion.

In addition, another technical object to be achieved by the present invention is to provide an electrocardiogram monitoring device with electrical stimulation function, which does not require additional biometric equipment or electrical stimulation equipment, in addition to an electrocardiogram monitoring device, and thus, can improve the accuracy of electrocardiogram monitoring while achieving the user's electrocardiogram measurement module and electrical stimulation based thereon through a single device for continuous monitoring and biofeedback in daily life.

The technical objects to be achieved by the present invention are not limited to the technical objects mentioned above, and other technical objects not mentioned may be clearly understood by those skilled in the art from the following descriptions.

### [Technical Solution]

The invention is defined by the independent claims. In order to achieve the technical objects, an electrocardiogram monitoring device according to claim 1 is provided.

According to an embodiment of the present invention, the electrical stimulation may include transcutaneous electrical nerve stimulation (TENS).

According to an embodiment of the present invention, the acquisition cycle may be 1 ms to 10ms.

According to an embodiment of the present invention, the electrical stimulation module may provide the electrical stimulation with a pulse width of 150 to 400 µs and a frequency of 15 to 25 Hz to the user through the two or more electrode pads.

According to an embodiment of the present invention, the electrocardiogram measurement module may include a monitoring mode and a rest mode, in the monitoring mode, the electrocardiogram measurement module may acquire an electrocardiogram signal of the user at a measurement cycle timing of a predetermined pattern and measure an electrocardiogram, and in the rest mode, the electrocardiogram measurement module stops measuring the electrocardiogram, the control module may control the electrical stimulation module to output the electrical stimulation between the signal acquisition timings of the acquisition cycle based on the electrocardiogram measurement result for the user during the monitoring mode of the electrocardiogram measurement module.

According to an embodiment of the present invention, the electrocardiogram measurement module may digitally convert an electrocardiogram signal acquired within each signal acquisition cycle and generates an analog to digital conversion (ADC) signal, the control module may control the electrical stimulation module to output the electrical stimulation between the ADC signal generation timing and a next electrocardiogram signal acquisition timing based on the electrocardiogram measurement result for the user from the electrocardiogram measurement module.

According to an embodiment of the present invention, during the monitoring mode, when the electrocardiogram measurement result for the user from the electrocardiogram measurement module returns to a predetermined normal range, the control module may control the electrical stimulation module to stop providing the electrical stimulation to the user.

According to an embodiment of the present invention, the electrocardiogram measurement module may filter noise of an electrocardiogram signal by an adaptive noise canceller that uses an output signal of the electrical stimulation as a reference signal.

According to an embodiment of the present invention, the electrocardiogram monitoring device may include electrical stimulation function in which a straight line connecting the two or more electrodes for measuring the electrocardiogram and a straight line connecting the two or more electrode pads providing the electrical stimulation is configured to be arranged perpendicular to each other.

According to an embodiment of the present invention, a communication module that transmits and receives data with a user terminal or external system through a network may be further comprised.

### [Advantageous Effects]

An electrocardiogram monitoring device with electrical stimulation function according to an embodiment of the present invention integrates an electrocardiogram measurement module and an electrical stimulation module that provides biofeedback based on the module into one device, while alternatively providing electrocardiogram measurement timing and electrical stimulation output timing. Therefore, the device enables continuous electrocardiogram monitoring and biofeedback based on the monitoring in daily life without spatial constraints, while improving the accuracy of electrocardiogram monitoring by minimizing distortion of the electrocardiogram measurement signal.

The effects of the present disclosure are not limited to the above-mentioned effects, and it should be understood that the effects of the present disclosure include all effects that could be inferred from the configuration of the invention described in the detailed description of the invention or the appended claims.

### [Description of Drawings]

FIG. 1 shows a graph of a normal electrocardiogram (ECG) measurement measured by a conventional electrocardiogram monitoring device when no electrical stimulation is applied to a user.
FIG. 2 shows a graph of an electrocardiogram (ECG) measurement measured by the same conventional electrocardiogram monitoring device as that of FIG. 1 while applying electrical stimulation to a user equipped with an electrical stimulation device.
FIG. 3 is a block diagram schematically showing the functional configuration of an electrocardiogram monitoring device according to an embodiment of the present invention.
FIG. 4 is a graph showing comparison of the measurement timing and cycle for measuring the user's electrocardiogram by the electrocardiogram monitoring device according to an embodiment of the present invention in FIG. 3 and the output timing and cycle for outputting electrical stimulation to apply electrical stimulation to the user.
FIG. 5 shows a flowchart in which an electrocardiogram monitoring device according to an embodiment of the present invention reduces distortion of the signal by applying an adaptive noise canceller to an electrocardiogram measurement signal.
FIG. 6 shows a schematic diagram of an embodiment of the adaptive noise canceller used in FIG. 5.
FIG. 7 shows a schematic diagram schematically showing the configuration of an electrocardiogram monitoring device according to an embodiment of the present invention.
FIG. 8A shows a graph of electrocardiogram measured when applying electrical stimulation to the user's both hands in a state where a direction of connecting the LA and RA electrodes of an electrocardiogram monitoring device according to an embodiment of the present invention is positioned horizontally to a direction of connecting an electrical stimulation electrode pads of both hands.
FIG. 8B shows a graph of electrocardiogram measured when applying electrical stimulation to the user's both hands in a state where a direction of connecting the LA and RA electrodes of an electrocardiogram monitoring device according to an embodiment of the present invention is positioned at 45° with a direction of connecting electrical stimulation electrode pads of both hands.
FIG. 8C shows a graph of electrocardiogram measured when applying electrical stimulation to the user's both hands in a state where a direction of connecting the LA and RA electrodes of an electrocardiogram monitoring device according to an embodiment of the present invention is positioned perpendicular to the direction of connecting electrical stimulation electrode pads of both hands.

### [Mode for Invention]

Hereinafter, the present invention will be explained with reference to the accompanying drawings. The present invention, however, may be modified in various different ways, and should not be construed as limited to the embodiments set forth herein. Also, in order to clearly explain the present invention, portions that are not related to the present invention are omitted, and like reference numerals are used to refer to like elements throughout.

Throughout the specification, it will be understood that when an element is referred to as being "connected (accessed, contacted, coupled) to" another element, this includes not only cases where the elements are "directly connected," but also cases where the elements are "indirectly connected" with another member therebetween. Also, it will also be understood that when a component "includes" an element, unless stated otherwise, this does not mean that other elements are excluded, but that other element may be further added.

Terms used herein may be merely to describe a certain embodiment, and may not be intended to limit the disclosure. The singular expressions may include plural expressions unless the context clearly dictates otherwise. In the specification, it will be further understood that the terms "comprise" and "include" specify the presence of stated features, integers, steps, operations, elements, components, or combinations thereof, but do not preclude in advance the possibility of the presence or addition of one or more other features, integers, steps, operations, elements, components, or combinations.

Hereinafter, an electrocardiogram monitoring device with electrical stimulation function according to an embodiment of the present invention will be described with reference to the drawings.

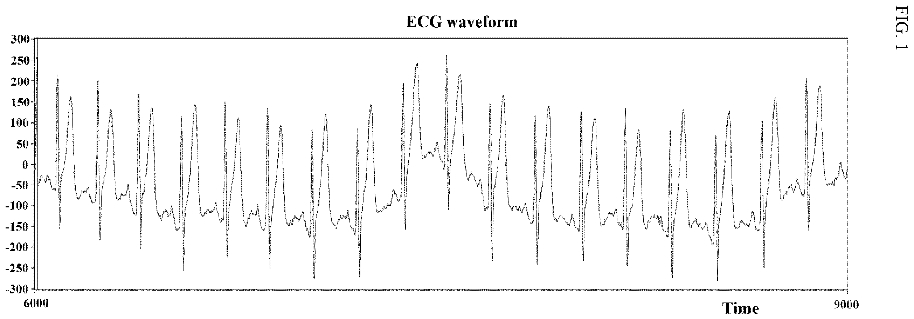

FIG. 1 shows a graph of a normal electrocardiogram (ECG) measurement measured by a conventional electrocardiogram monitoring device when no electrical stimulation is applied to a user. The graph in FIG. 1 is an electrocardiogram measurement graph of a healthy user who does not have a heart condition, and shows a graph in which no distortion occurs in the electrocardiogram measurement signal because electrical stimulation is not applied to the user.

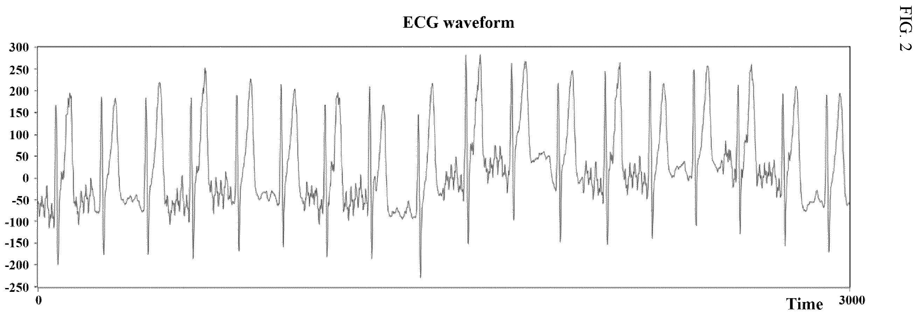

On the other hand, FIG. 2 shows a graph of an electrocardiogram (ECG) measurement measured by the same conventional electrocardiogram monitoring device as that of FIG. 1 while applying electrical stimulation to a user equipped with an electrical stimulation device. In the case of FIG. 2, although the electrocardiogram was measured by applying electrical stimulation by attaching a separate, independent electrical stimulation device to the back of the user's neck, where the effect of electrical stimulation on the electrocardiogram is relatively small, when compared to the normal electrocardiogram measurement graph in FIG. 1, it can be confirmed in the graph in FIG. 2 that distortion, such as noise, occurs in the electrocardiogram measurement signal when electrical stimulation is applied.

Recognizing this problem, the inventor has invented an electrocardiogram monitoring device, which is not limited to simply monitoring and analyzing electrocardiogram measurement results, but can immediately provide feedback, such as an alarm or light treatment, to the user through electrical stimulation when an abnormality is detected in the results even while the electrocardiogram monitoring device is operating in a monitoring mode, while maintaining monitoring accuracy by minimizing distortion of electrocardiogram measurement results due to the electrical stimulation.

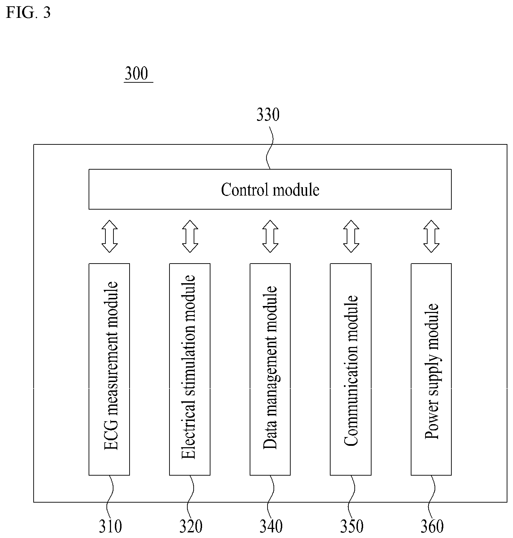

FIG. 3 is a block diagram schematically showing the functional configuration of an electrocardiogram monitoring device according to an embodiment of the present invention. Referring to FIG. 3, an ECG monitoring device 300 may include an ECG measurement module 310, an electrical stimulation module 320, and a control module 330. Additionally, the ECG monitoring device 300 may further include a data management module 340, a communication module 350, and a power supply module 360.

The ECG measurement module 310 may measure the user's electrocardiogram at a predetermined electrocardiogram signal acquisition cycle, and may include a signal acquisition unit including two or more ECG measurement electrodes for ECG measurement, and a signal analysis unit that analyzes the ECG signal acquired from the signal acquisition unit and provides status diagnosis results and/or ECG measurement signal data to the user as an analysis result. The ECG measurement module 310 may include a monitoring mode and a rest mode. In the monitoring mode, the ECG measurement module 310 may measure the user's ECG at a measurement cycle timing of a predetermined pattern through the signal acquisition unit. In the rest mode, ECG measurement of the ECG measurement module 310 may be stopped.

During the monitoring mode, the ECG measurement module 310 may acquire an ECG signal from the signal acquisition unit at a cycle of 1 ms to 10 ms or a frequency of 100 Hz to 1000 Hz, but is not limited to this cycle. The ECG measurement module 310 may convert an analog signal into a digital signal immediately after acquiring the user's ECG signal at the cycle from the signal acquisition unit to generate an analog to digital conversion (ADC) signal. The generation cycle interval of the ADC signal is the same as the cycle interval for acquiring the ECG signal, and may be preferably 1 ms to 10 ms, more preferably 1 ms to 2 ms, but is not limited thereto. The signal analysis unit may receive the ADC signal generated by the signal acquisition unit to analyze the user's cardiac signs, and the analysis result of the ADC signal may be transmitted to the control module 330, data management module 340, and/or communication module 350.

The electrical stimulation module 320 may include an electrical stimulation output unit that outputs the electrical stimulation and a stimulation pad including two or more electrical stimulation electrodes that are electrically connected to the electrical stimulation output unit and are in contact with the user's skin to apply the electrical stimulation. The electrical stimulation output unit may output at least one electrical stimulation pattern, and the electrical stimulation pattern may be set by changing at least one of electrical stimulation timing, time interval, frequency, and output intensity. The electrical stimulation module 420 may preferably apply transcutaneous electrical nerve stimulation (TENS) through the two or more electrical stimulation electrodes, but is not limited thereto. In addition, the electrical stimulation may be, but is not limited to, electrical stimulation with a pulse width of 150 to 400 µs and a frequency of 15 to 25 Hz. The electrical stimulation module 320 may include a first output mode that outputs the electrical stimulation to the user during the monitoring mode of the ECG measurement module 310, and a second output mode that outputs the electrical stimulation to the user after switching to the rest mode of the ECG measurement module 310. In the first output mode, the electrical stimulation module 320 may be configured to apply a plurality of electrical stimulation patterns based on the measurement results of the ECG measurement module 310.

The control module 330 may transmit and receive data with the ECG measurement module 310 and the electrical stimulation module 320 and control the operations of the ECG measurement module 310 and electrical stimulation module 320. The control module 330 may control the electrical stimulation module 320 to output and provide the electrical stimulation to the user in order to provide a warning to the user or adjust the user's condition based on the status diagnosis results provided by the ECG measurement module 310. In one embodiment of the present invention, when a status diagnosis result indicating that the user's heart condition is 'abnormal' is provided while the monitoring mode of the ECG measurement module 310 is operating, the control module 330 may control the electrical stimulation module 320 to provide one electrical stimulation of at least one electrical stimulation pattern to the user through this electrical stimulation electrode.

The control module 330 may control the type of electrical stimulation pattern provided by the electrical stimulation module 320 based on the heart rate determined by the ECG measurement module 310 or the determination of the user's heart condition according to the analysis. Based on the ECG measurement results, the control module 330 may control the electrical stimulation module 320 to provide the electrical stimulation to the user only during the interval between signal acquisition timings in the ECG signal acquisition cycle, that is, the time interval during which ECG measurement is not performed. In addition, the control module 330 may reduce distortion in the measured ECG signal by controlling the timing of the electrical stimulation output of the electrical stimulation module 320 to intersect entirely or partially with the ECG signal acquisition timing of the ECG measurement module 310, based on the ECG measurement result of the ECG measurement module 310. In one embodiment of the present invention, the control module 330 may control the electrical stimulation module to output the electrical stimulation between the ADC signal generation timing and a next electrocardiogram signal acquisition timing based on the electrocardiogram measurement result for the user from the ECG measurement module 330. During the monitoring mode, when the ECG measurement result for the user from the ECG measurement module 310 returns to a predetermined normal range, the control module 330 may control the electrical stimulation module 320 to stop providing the electrical stimulation to the user.

The data management module 340 may store and process data generated from at least one of the ECG measurement module 310, electrical stimulation module 320, and control module 330.

The communication module 350 allows the ECG monitoring device 300 to transmit and receive data with a user terminal such as a smartphone or tablet or an external system through a network. Examples of networks include 3rd Generation Partnership Project (3GPP) network, Long Term Evolution (LTE) network, 5G network, World Interoperability for Microwave Access (WIMAX) network, wired and wireless Internet, Local Area Network (LAN), and Wireless Local Area Network (Wireless LAN), Wide Area Network (WAN), Personal Area Network (PAN), Bluetooth network, Wifi network, Near Field Communication (NFC) network, satellite broadcasting network, analog broadcasting network, Digital Multimedia Broadcasting (DMB) networks, etc., but are not limited thereto.

The power supply module 360 may include at least one of a battery unit capable of charging or discharging electricity, and a power connection unit capable of supplying power by connecting to a commercial power grid.

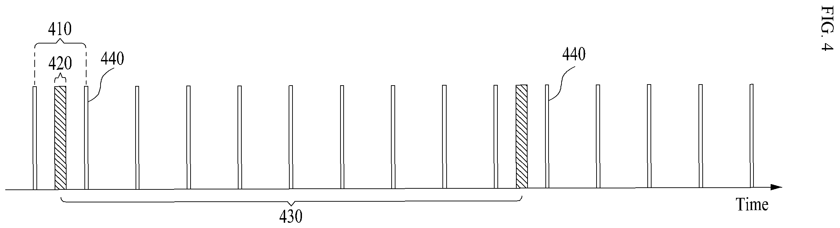

FIG. 4 is a graph showing comparison of the measurement timing and cycle for measuring the user's electrocardiogram by the electrocardiogram monitoring device according to an embodiment of the present invention in FIG. 3 and the output timing and cycle for outputting electrical stimulation to apply electrical stimulation to the user.

In one embodiment of the present invention shown in FIG. 4, when an ECG signal acquisition cycle 410 is 2 ms, a pulse width 420 of the applied electrical stimulation is 200 µs, and a pulse cycle 430 is 20 ms, the electrical stimulation output timing occurs at a location other than the ECG measurement timing. That is, the electrical stimulation is applied between the nth ECG acquisition timing and the n+1th ECG acquisition timing. That is, during the monitoring mode in which the ECG measurement module 310 measures the ECG at a predetermined ECG signal acquisition cycle, the electrical stimulation module controls the electrical stimulation output timing to be formed for the user only during a period that does not overlap with the ECG measurement timing within one cycle, thereby minimizing distortion caused by the electrical stimulation during the ECG measurement. In one embodiment of the present invention, the electrical stimulation output timing may be controlled to entirely or partially intersect temporally with the ECG acquisition timing.

However, since the ECG signal acquisition cycle in the monitoring mode in one embodiment of the present invention is very short, for example, about 1 to 10 ms, even if the timing of the electrical stimulation output is temporally arranged between the ECG acquisition timings so as not to overlap with the ECG acquisition timing, in the case of the ECG signal 440 measured immediately after the application of the electrical stimulation, slight residual distortion may occur due to the applied electrical stimulation.

Therefore, in the ECG monitoring device 300 of an embodiment of the present invention, the signal analysis unit of the ECG measurement module 310 filters the ECG signal acquired from the signal acquisition unit by an adaptive noise canceller (ANC) 500 and then analyze the signal, thereby further minimizing signal distortion.

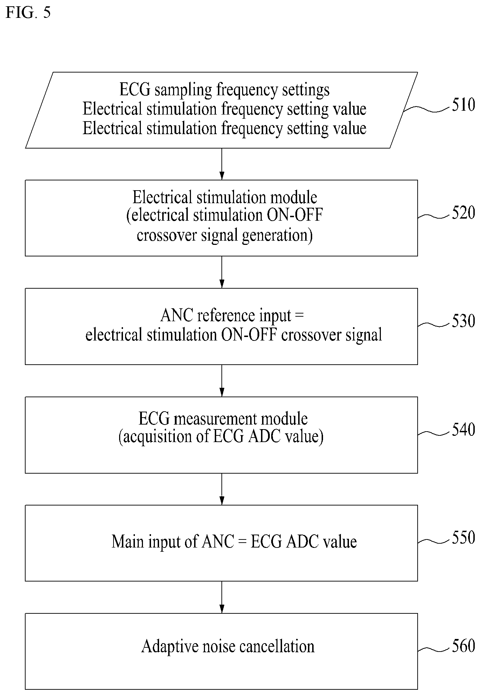

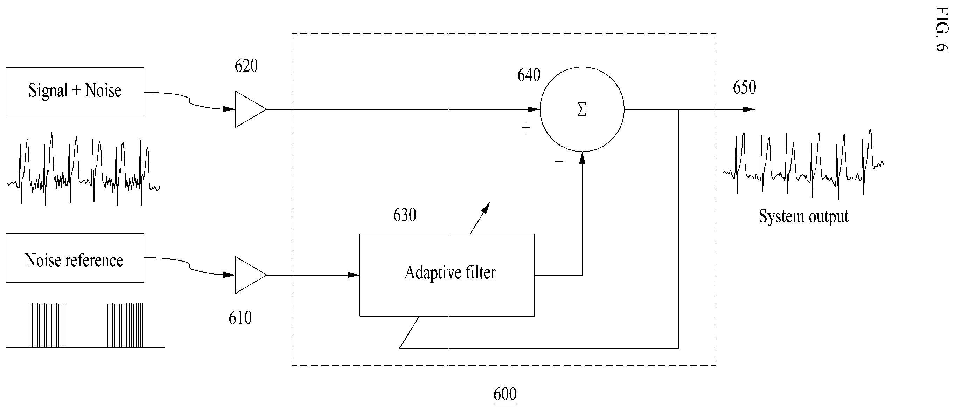

FIG. 5 shows a flowchart of a process in which the ECG monitoring device 300 according to an embodiment of the present invention reduces distortion of the signal by applying the adaptive noise canceller to an electrocardiogram measurement signal. FIG. 6 shows a schematic diagram of an embodiment of the adaptive noise canceller 500 used in FIG. 5.

Specifically, referring to FIGS. 5 and 6, first, in step S510, the ECG sampling frequency of the ECG measurement module 310 of the ECG monitoring device 300 and the electrical stimulation frequency and pulse width of the electrical stimulation module 320 are set by the control module 330, and the signal acquisition unit of the ECG measurement module 310 measures the ECG signal at a predetermined cycle.

In step S520, when the signal analysis unit determines that the ECG signal of the ECG measurement module is abnormal, the control module 330 outputs an electrical stimulation on-off crossover signal at the timing of a time period during which ECG signal measurement is not performed within the ECG signal acquisition cycle so that the ECG signal acquisition timing and/or ADC signal generation timing of the ECG measurement module 310 do not overlap, and thus, controls the electrical stimulation to be applied to the skin of the user.

In step S530, the control module 330 may input the on-off crossover signal of the electrical stimulation as a reference input 620 of the adaptive noise canceller 500, where the reference input 620 may include the reference to noise caused by the electrical stimulation output.

In step S540, after the on-off crossover signal of the electrical stimulation is output, the ECG measurement module 310 acquires the user's ECG signal again through the signal acquisition unit and digitally converts it to generate an ADC signal. The ADC signal may be an ECG waveform in which minute residual noise and/or distortion is generated due to the electrical stimulation. In step S550, the ECG measurement module 310 inputs the ECG ADC signal as the main input 620 of the adaptive noise controller 600, and in step S560, the adaptive noise canceller 600 cancels the noise generated by the electrical stimulation by filtering the input ECG ADC signal with reference to the reference signal.

The adaptive noise canceller 600 according to an embodiment of the present invention may include an adaptive filter 630. In a general time domain, the input signal is delayed by a time delay element to generate N sequences, each of which is multiplied by a weight and then added again (640) to generate an output signal (650). A method in which these output signals may be compared with a desired reference input signal to obtain an error and this error is used to change the weight (630) may be used.

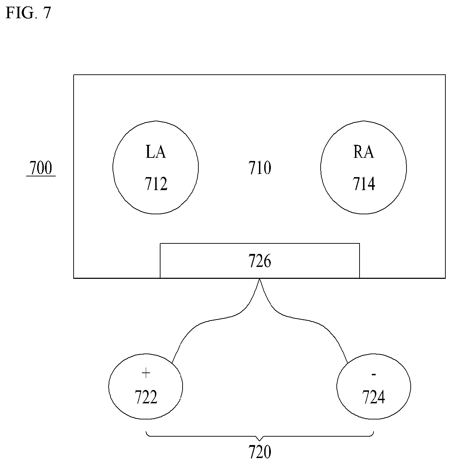

FIG. 7 shows a schematic diagram schematically showing the configuration of an electrocardiogram monitoring device according to an embodiment of the present invention. In FIG. 7, components corresponding to the control module 330, data management module 340, communication module 350, and power supply module 360 in FIG. 3 are not shown for simplicity. Referring to FIG. 7, an ECG monitoring device 700 with electrical stimulation function according to an embodiment of the present invention may include an ECG measurement module 710 and an electrical stimulation module 720.

The ECG measurement module 710 may include a signal acquisition unit including two left electrodes (LA) 712 and right electrodes (RA) 714 for ECG measurement, and a signal analysis unit that analyzes the signal provided by the signal acquisition unit. When the signal analysis unit determines that a waveform that satisfies predetermined characteristic conditions exists within the waveform of the ECG signal acquired by the signal acquisition unit, it may first determine that a signal corresponding to the waveform that satisfies the predetermined characteristic conditions is a suspected abnormal ECG signal. The predetermined characteristic condition may be a condition for a characteristic in which a signal belonging to a threshold range occurs during a predetermined time.

The electrical stimulation module 720 may include an electrical stimulation electrode pad unit including two electrical stimulation electrode pads 722 and 724 that provide a positive electrode (+) and a negative electrode (-) respectively for contacting the user's skin and applying electrical stimulation, and an electrical stimulation output 722 that outputs electrical stimulation to the electrical stimulation electrode pad unit according to control signals from the control module. The two electrical stimulation electrode pads 722 and 724 may be attached to the right and left hands, respectively, for example.

When an angle between a straight line connecting the two electrodes for measuring the ECG and a straight line connecting the two electrical stimulation electrode pads for outputting the electrical stimulation changes, a degree to which noise and/or distortion is generated in the ECG measurement signal of the electrical stimulation applied to the user is different. Thus, two electrodes for measuring the ECG and two electrical stimulation electrode pads for outputting the electrical stimulation may be arranged within the ECG monitoring device 700 to minimize this effect. For example, in the ECG monitoring device according to an embodiment of the present invention, a straight line connecting the two electrodes for measuring ECG and a straight line connecting the two electrical stimulation electrode pads for outputting the electrical stimulation may be arranged perpendicular to each other in order to minimize distortion of the ECG measurement signal.

However, in the ECG monitoring device 700 according to an embodiment of the present invention in FIG. 7, when an angle of the straight line connecting the two electrodes for measuring ECG and the straight line connecting the two electrical stimulation electrode pads for outputting the electrical stimulation is changed, there is a possibility that a problem may arise in which the degree of influence of distortion on the ECG measurement signal of the electrical stimulation applied to the user may be different.

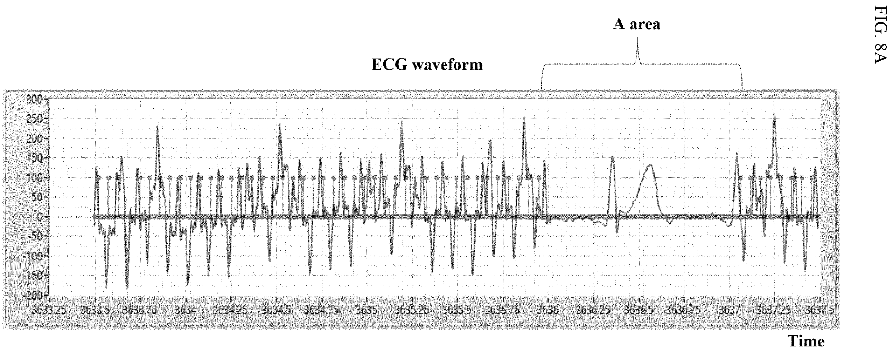

FIG. 8A shows a graph of ECG measured by the two electrical stimulation electrode pads 722 and 724 of the ECG monitoring device according to an embodiment of the present invention of FIG. 7 when applying electrical stimulation to the user's both hands in a state where a direction of connecting the LA and RA electrodes 712 and 714 of is positioned horizontally to a direction of connecting the electrical stimulation electrode pads 722 and 724 of both hands. In the graph of FIG. 8A, compared to a section (area A) in which electrical stimulation is not applied to the user, it can be seen that the ECG waveform resulting from the ECG measurement has very severe distortion and noise in the time section (area A) where electrical stimulation is applied simultaneously with the ECG measurement.

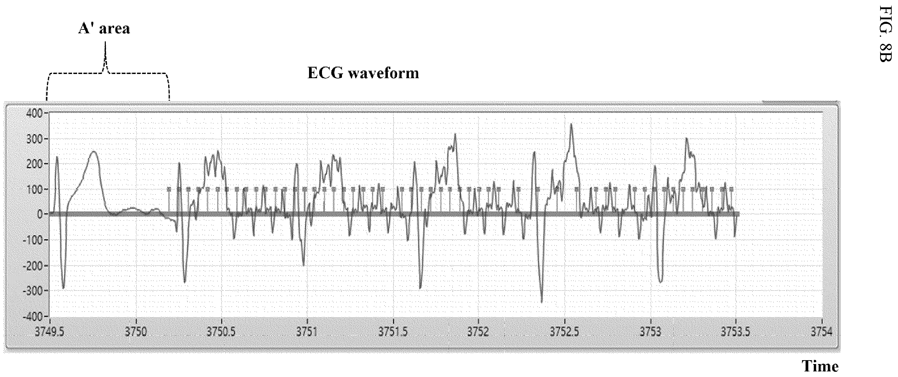

FIG. 8B shows a graph of ECG measured by the two electrical stimulation electrode pads 722 and 724 of the ECG monitoring device according to an embodiment of the present invention of FIG. 7 when applying electrical stimulation to the user's both hands in a state where a direction of connecting the LA and RA electrodes 712 and 714 is positioned at 45° with a direction of connecting electrical stimulation electrode pads of both hands. In the graph of FIG. 8B, compared to a section (area A') in which electrical stimulation is not applied to the user, it can be seen that there is considerable distortion and noise in the ECG waveform that is the result of the ECG measurement in the time section in which the electrical stimulation is applied simultaneously with the ECG measurement. However, when compared to FIG. 8A, the overall regularity of the ECG pattern appears to be not disrupted.

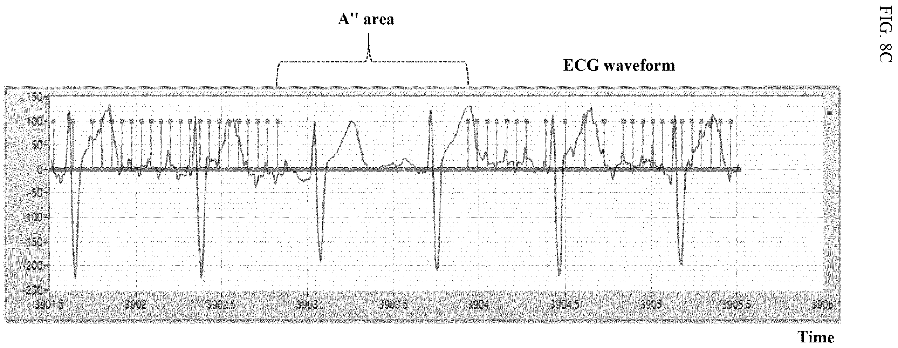

FIG. 8C shows a graph of electrocardiogram measured by the two electrical stimulation electrode pads 722 and 724 of the ECG monitoring device according to an embodiment of the present invention of FIG. 7 when applying electrical stimulation to the user's both hands in a state where a direction of connecting the LA and RA electrodes 712 and 714 is positioned perpendicular to the direction of connecting electrical stimulation electrode pads of both hands. In the graph of FIG. 8C, compared to a section (area A") in which electrical stimulation is not applied to the user, it can be confirmed that distortion and noise in the ECG waveform are significantly reduced in the time section where electrical stimulation is applied simultaneously with ECG measurement, and only local distortion and noise appear.

Therefore, in the ECG monitoring device with electrical stimulation function according to an embodiment of the present invention, in order for the user's ECG measurement signal to be analyzed by minimizing the influence of noise or distortion due to the application of electrical stimulation, more preferably, the straight line connecting the two electrodes for the ECG measurement within the ECG monitoring device 600 and the straight line connecting the two electrical stimulation electrode pads that output the electrical stimulation can be arranged to be perpendicular to each other.

The description of the present invention is used for illustration and those skilled in the art will understand that the present invention can be easily modified to other detailed forms. Therefore, the aforementioned exemplary embodiments are all illustrative in all aspects and are not limited. For example, each component described as a single type may be implemented to be distributed and similarly, components described to be distributed may also be implemented in a combined form.

The scope of the present invention is to be defined by the scope of claims provided below.

### [Industrial Applicability]

The present invention may provide an electrocardiogram monitoring device with electrical stimulation function which integrates an electrocardiogram measurement module and an electrical stimulation module that provides biofeedback based on the module into one device, while alternatively providing electrocardiogram measurement timing and electrical stimulation output timing. Therefore, the device enables continuous electrocardiogram monitoring and biofeedback based on the monitoring in daily life without spatial constraints, while improving the accuracy of electrocardiogram monitoring by minimizing distortion of the electrocardiogram measurement signal.

## Claims

1. An electrocardiogram monitoring device (300; 700), comprising:
an electrocardiogram measurement module (310; 710), which includes two or more electrodes (712, 714) for electrocardiogram measurement and measures the electrocardiogram of a user at a predetermined electrocardiogram signal acquisition cycle;
an electrical stimulation module (320; 720) including two or more electrode pads (722, 724), which make contact with the skin of the user and provide electrical stimulation, wherein the two or more electrode pads (722, 724) are separated from the two or more electrodes (712, 714) for electrocardiogram measurement; and
a control module (330) for controlling operations of the electrocardiogram measurement module (310; 710) and electrical stimulation module (320; 720),
wherein the control module (300) controls the electrical stimulation module (320; 720) to output the electrical stimulation between signal acquisition timings of the acquisition cycle based on an electrocardiogram measurement result for the user from the electrocardiogram measurement module (310; 710).

2. The electrocardiogram monitoring device (300; 700) of claim 1, wherein the electrical stimulation includes transcutaneous electrical nerve stimulation (TENS).

3. The electrocardiogram monitoring device (300; 700) of claim 1, wherein the acquisition cycle is 1 ms to 10 ms.

4. The electrocardiogram monitoring device (300; 700) of claim 1, wherein the electrical stimulation module (320; 720) provides the electrical stimulation with a pulse width of 150 to 400 µs and a frequency of 15 to 25 Hz to the user through the two or more electrode pads (722, 724).

5. The electrocardiogram monitoring device (300; 700) of claim 1, wherein the electrocardiogram measurement module (310; 710) includes a monitoring mode and a rest mode, in the monitoring mode, the electrocardiogram measurement module (310; 710) acquires an electrocardiogram signal of the user at a measurement cycle timing of a predetermined pattern and measures electrocardiogram, and in the rest mode, the electrocardiogram measurement module stops measuring the electrocardiogram,
wherein the control module (330) controls the electrical stimulation module (320; 720) to output the electrical stimulation between the signal acquisition timings of the acquisition cycle based on the electrocardiogram measurement result for the user during the monitoring mode of the electrocardiogram measurement module (310; 710).

6. The electrocardiogram monitoring device (300; 700) of claim 1, wherein the electrocardiogram measurement module (310; 710) digitally converts an electrocardiogram signal acquired within each signal acquisition cycle and generates an analog to digital conversion (ADC) signal,
wherein the control module (330) controls the electrical stimulation module (320; 720) to output the electrical stimulation between the ADC signal generation timing and a next electrocardiogram signal acquisition timing based on the electrocardiogram measurement result for the user from the electrocardiogram measurement module (310, 710).

7. The electrocardiogram monitoring device (300; 700) of claim 5, wherein during the monitoring mode, when the electrocardiogram measurement result for the user from the electrocardiogram measurement module (310; 710) returns to a predetermined normal range, the control module (330) controls the electrical stimulation module (320; 720) to stop providing the electrical stimulation to the user.

8. The electrocardiogram monitoring device (300; 700) of claim 1, wherein the electrocardiogram measurement module (310; 710) filters noise of an electrocardiogram signal by an adaptive noise canceller that uses an output signal of the electrical stimulation module (320, 720) as a reference signal.

9. The electrocardiogram monitoring device of claim 1, further comprising a communication module that transmits and receives data with a user terminal or external system through a network.

10. A method for operating an electrocardiogram monitoring device (300; 700), the method comprising the steps of
providing an electrocardiogram monitoring device (300; 700) according to claim 1
arranging the two or more electrodes (712, 714) for measuring the electrocardiogram and the two or more electrode pads (722, 724) providing the electrical stimulation at a user in such way that a straight line connecting the two or more electrodes (712, 714) for measuring the electrocardiogram and a straight line connecting the two or more electrode pads (722, 724) providing the electrical stimulation are arranged perpendicular to each other.

## Patentansprüche

1. Elektrokardiogramm-Überwachungsvorrichtung (300; 700), die folgende Merkmale aufweist:
ein Elektrokardiogramm-Messmodul (310; 710), das zwei oder mehr Elektroden (712, 714) zur Elektrokardiogramm-Messung aufweist und das Elektrokardiogramm eines Benutzers bei einem vorbestimmten Elektrokardiogramm-Signalerfassungszyklus misst;
ein elektrisches Stimulationsmodul (320; 720), das zwei oder mehr Elektrodenpads (722, 724) aufweist, die mit der Haut des Benutzers Kontakt herstellen und eine elektrische Stimulation bereitstellen, wobei die zwei oder mehr Elektrodenpads (722, 724) von den zwei oder mehr Elektroden (712, 714) zur Elektrokardiogramm-Messung getrennt sind; und
ein Steuermodul (330) zum Steuern von Operationen des Elektrokardiogramm-Messmoduls (310; 710) und des elektrischen Stimulationsmoduls (320; 720),
wobei das Steuermodul (300) das elektrische Stimulationsmodul (320; 720) steuert, um die elektrische Stimulation zwischen Signalerfassungszeitgebungen des Erfassungszyklus basierend auf einem Elektrokardiogramm-Messergebnis für den Benutzer von dem Elektrokardiogramm-Messmodul (310; 710) auszugeben.

2. Elektrokardiogramm-Überwachungsvorrichtung (300; 700) gemäß Anspruch 1, wobei die elektrische Stimulation transkutane elektrische Nervenstimulation (TENS) aufweist.

3. Elektrokardiogramm-Überwachungsvorrichtung (300; 700) gemäß Anspruch 1, wobei der Erfassungszyklus 1 ms bis 10 ms beträgt.

4. Elektrokardiogramm-Überwachungsvorrichtung (300; 700) gemäß Anspruch 1, wobei das elektrische Stimulationsmodul (320; 720) die elektrische Stimulation mit einer Pulsbreite von 150 bis 400 µs und einer Frequenz von 15 bis 25 Hz für den Benutzer durch die zwei oder mehr Elektrodenpads (722, 724) bereitstellt.

5. Elektrokardiogramm-Überwachungsvorrichtung (300; 700) gemäß Anspruch 1, wobei das Elektrokardiogramm-Messmodul (310; 710) einen Überwachungsmodus und einen Ruhemodus aufweist, wobei in dem Überwachungsmodus das Elektrokardiogramm-Messmodul (310; 710) ein Elektrokardiogramm-Signal des Benutzers zu einer Messzyklus-Zeitgebung eines vorbestimmten Musters erfasst und ein Elektrokardiogramm misst, und in dem Ruhemodus das Elektrokardiogramm-Messmodul ein Messen des Elektrokardiogramms stoppt,
wobei das Steuermodul (330) das elektrische Stimulationsmodul (320; 720) steuert, um die elektrische Stimulation zwischen den Signalerfassungszeitgebungen des Erfassungszyklus basierend auf dem Elektrokardiogramm-Messergebnis für den Benutzer während des Überwachungsmodus des Elektrokardiogramm-Messmoduls (310; 710) auszugeben.

6. Elektrokardiogramm-Überwachungsvorrichtung (300; 700) gemäß Anspruch 1, wobei das Elektrokardiogramm-Messmodul (310; 710) ein Elektrokardiogramm-Signal, das innerhalb jedes Signalerfassungszyklus erfasst wird, digital umwandelt und ein Analog-Digital-Umwandlungs(ADC)-Signal erzeugt,
wobei das Steuermodul (330) das elektrische Stimulationsmodul (320; 720) steuert, um die elektrische Stimulation zwischen der ADC-Signalerzeugungszeitgebung und einer nächsten Elektrokardiogramm-Signalerfassungszeitgebung basierend auf dem Elektrokardiogramm-Messergebnis für den Benutzer von dem Elektrokardiogramm-Messmodul (310, 710) auszugeben.

7. Elektrokardiogramm-Überwachungsvorrichtung (300; 700) gemäß Anspruch 5, wobei während des Überwachungsmodus, wenn das Elektrokardiogramm-Messergebnis für den Benutzer von dem Elektrokardiogramm-Messmodul (310; 710) in einen vorbestimmten Normalbereich zurückkehrt, das Steuermodul (330) das elektrische Stimulationsmodul (320; 720) steuert, um ein Bereitstellen der elektrischen Stimulation für den Benutzer zu stoppen.

8. Elektrokardiogramm-Überwachungsvorrichtung (300; 700) gemäß Anspruch 1, wobei das Elektrokardiogramm-Messmodul (310; 710) Rauschen eines Elektrokardiogramm-Signals durch einen adaptiven Rauschunterdrücker filtert, der ein Ausgangssignal des elektrischen Stimulationsmoduls (320, 720) als ein Referenzsignal verwendet.

9. Elektrokardiogramm-Überwachungsvorrichtung gemäß Anspruch 1, die ferner ein Kommunikationsmodul aufweist, das Daten mit einem Benutzerendgerät oder einem externen System über ein Netzwerk sendet und empfängt.

10. Verfahren zum Betreiben einer Elektrokardiogramm-Überwachungsvorrichtung (300; 700), wobei das Verfahren folgende Schritte aufweist:
Bereitstellen einer Elektrokardiogramm-Überwachungsvorrichtung (300; 700) gemäß Anspruch 1,
Anordnen der zwei oder mehr Elektroden (712, 714) zum Messen des Elektrokardiogramms und der zwei oder mehr Elektrodenpads (722, 724), die die elektrische Stimulation an einem Benutzer bereitstellen, derart, dass eine gerade Linie, die die zwei oder mehr Elektroden (712, 714) zum Messen des Elektrokardiogramms verbindet, und eine gerade Linie, die die zwei oder mehr Elektrodenpads (722, 724) verbindet, die die elektrische Stimulation bereitstellen, senkrecht zueinander angeordnet sind.

## Revendications

1. Dispositif de surveillance électrocardiographique (300 ; 700), comprenant :
un module de mesure électrocardiographique (310 ; 710), qui inclut deux ou plusieurs électrodes (712, 714) destinées à la mesure électrocardiographique et qui mesure l'électrocardiogramme d'un utilisateur selon un cycle prédéterminé d'acquisition de signal électrocardiographique ;
un module de stimulation électrique (320 ; 720) incluant deux ou plusieurs plots d'électrodes (722, 724) qui entrent en contact avec la peau de l'utilisateur et fournissent une stimulation électrique, dans lequel les deux ou plusieurs plots d'électrodes (722, 724) sont séparés des deux ou plusieurs électrodes (712, 714) destinées à la mesure électrocardiographique ; et
un module de commande (330) destiné à commander le fonctionnement du module de mesure électrocardiographique (310 ; 710) et du module de stimulation électrique (320 ; 720),
dans lequel le module de commande (300) commande le module de stimulation électrique (320 ; 720) afin qu'il délivre la stimulation électrique entre les instants d'acquisition de signaux du cycle d'acquisition sur la base d'un résultat de mesure électrocardiographique pour l'utilisateur provenant du module de mesure électrocardiographique (310 ; 710).

2. Dispositif de surveillance électrocardiographique (300 ; 700) selon la revendication 1, dans lequel la stimulation électrique inclut une stimulation nerveuse électrique transcutanée (TENS).

3. Dispositif de surveillance électrocardiographique (300 ; 700) selon la revendication 1, dans lequel le cycle d'acquisition est compris entre 1 ms et 10 ms.

4. Dispositif de surveillance électrocardiographique (300 ; 700) selon la revendication 1, dans lequel le module de stimulation électrique (320 ; 720) délivre la stimulation électrique, dont la largeur d'impulsion est comprise entre 150 et 400 µs et dont la fréquence est comprise entre 15 et 25 Hz, à l'utilisateur, par l'intermédiaire des deux ou plusieurs plots d'électrodes (722, 724).

5. Dispositif de surveillance électrocardiographique (300 ; 700) selon la revendication 1, dans lequel le module de mesure électrocardiographique (310 ; 710) inclut un mode surveillance et un mode repos ; en mode surveillance, le module de mesure électrocardiographique (310 ; 710) acquiert un signal électrocardiographique de l'utilisateur selon un instant de cycle de mesure d'un schéma prédéterminé et mesure l'électrocardiogramme, et en mode repos, le module de mesure électrocardiographique cesse de mesurer l'électrocardiogramme,
dans lequel le module de commande (330) commande le module de stimulation électrique (320 ; 720) afin qu'il délivre la stimulation électrique entre les instants d'acquisition de signaux du cycle d'acquisition sur la base du résultat de mesure électrocardiographique pour l'utilisateur pendant le mode surveillance du module de mesure électrocardiographique (310 ; 710).

6. Dispositif de surveillance électrocardiographique (300 ; 700) selon la revendication 1, dans lequel le module de mesure électrocardiographique (310 ; 710) convertit numériquement un signal électrocardiographique acquis au cours de chaque cycle d'acquisition de signal et génère un signal de conversion analogique-numérique (ADC),
dans lequel le module de commande (330) commande le module de stimulation électrique (320 ; 720) afin qu'il délivre la stimulation électrique entre l'instant de génération du signal du convertisseur analogique-numérique (ADC) et un instant suivant d'acquisition du signal électrocardiographique, sur la base du résultat de la mesure électrocardiographique pour l'utilisateur provenant du module de mesure électrocardiographique (310, 710).

7. Dispositif de surveillance électrocardiographique (300 ; 700) selon la revendication 5, dans lequel, en mode surveillance, lorsque le résultat de la mesure électrocardiographique pour l'utilisateur, provenant du module de mesure électrocardiographique (310 ; 710), revient à une plage normale prédéterminée, le module de commande (330) commande le module de stimulation électrique (320 ; 720) afin qu'il cesse de délivrer la stimulation électrique à l'utilisateur.

8. Dispositif de surveillance électrocardiographique (300 ; 700) selon la revendication 1, dans lequel le module de mesure électrocardiographique (310 ; 710) filtre le bruit d'un signal électrocardiographique à l'aide d'un suppresseur de bruit adaptatif qui utilise un signal de sortie du module de stimulation électrique (320, 720) comme signal de référence.

9. Dispositif de surveillance électrocardiographique selon la revendication 1, comprenant en outre un module de communication qui transmet et reçoit des données avec un terminal utilisateur ou un système externe par l'intermédiaire d'un réseau.

10. Procédé de fonctionnement d'un dispositif de surveillance électrocardiographique (300 ; 700), le procédé comprenant les étapes consistant à
fournir un dispositif de surveillance électrocardiographique (300 ; 700) selon la revendication 1
la disposition des deux ou plusieurs électrodes (712, 714) destinées à mesurer l'électrocardiogramme et des deux ou plusieurs plots d'électrodes (722, 724) fournissant la stimulation électrique à un utilisateur de telle sorte qu'une ligne droite reliant les deux ou plusieurs électrodes (712, 714) destinées à mesurer l'électrocardiogramme et une ligne droite reliant les deux ou plusieurs plots d'électrodes (722, 724) fournissant la stimulation électrique soient disposées perpendiculairement l'une à l'autre.
